# EUROPEAN PATENT SPECIFICATION

(11) **EP 3 210 612 B1**
(45) Date of publication and mention of the grant of the patent: **21.11.2018**
(21) Application number: 17156432.1
(22) Date of filing: 16.02.2017
(51) Int. Cl.: A61K 35/742, A61P 17/02, A61P 31/00, A61P 31/04

(54) **SYNBIOTIC PREPARATION**
SYNBIOTISCHE ZUBEREITUNG
PRÉPARATION SYNBIOTIQUE

(30) Priority: 26.02.2016 BE 201605140
(43) Date of publication of application: 30.08.2017
(73) Proprietor: Chrisal NV, 3920 Lommel (BE)
(72) Inventor: TEMMERMAN, Robin, B-9160 Lokeren (BE); GIELEN, Corrie, B-3900 Overpelt (BE)
(74) Representative: IPLodge bvba

(56) References cited:
- EP-A1- 0 975 227
- WO-A1-2016/022779
- WO-A2-2011/022790
- US-A1- 2012 076 864
- AL-GHAZZEWI F H ET AL: "Impact of prebiotics and probiotics on skin health", BENEFICIAL MICROBES, vol. 5, no. 2, 2014, pages 99-107, XP009185514, ISSN: 1876-2883, DOI: 10.3920/BM2013.0040 [retrieved on 2014-02-28]

## Description

### Field of the invention

The invention relates to compositions containing prebiotics and probiotics for application to intact or damaged skin or tissue.

### Introduction

Recent reviews of the scientific literature [Baquerizo et al. (2014) J. Am. Acad Dermatol. 71(4): 814-21; Sonal Sekhar et al. (2014) Med. Hypotheses 82(1): 86-8; Sikorska et al. (2013) Med. Hypotheses 82(1): 86-8] indicate that the use of probiotics and prebiotics may have favourable effects in treatment of skin disorders or wound infections. However, there have been no studies in the scientific literature to date on the topical use of probiotics or prebiotics in humans. All of the limited number of studies available have used *in vitro* or mouse models, and have often used cell-free lysates of probiotic cultures. It is known from the scientific literature [Gungor et al. (2015) Benef. Microbes 6(5): 647-56] that application of non-pathogenic bacteria in the mouth and on prostheses can cause a reduction in infections, caries, halitosis, and other oral problems.

The scientific literature on the use of probiotics and prebiotics to treat skin conditions or wound infections primarily concerns the oral ingestion of *Lactobacillus* and *Bifidobacterium* preparations, which have a possible effect of strengthening the skin via internal stimulation of the immune system.

Based on the scientific literature, therefore, it has not been possible to produce probiotic or prebiotic compositions having a proven action on skin conditions and wound infections via topical application.

Patents teach that probiotics can be used in combating harmful bacteria, and that oral or topical administration of probiotics or cell extracts thereof can have a favourable effect in some skin conditions. WO 2006125283 teaches that the colonizing of surfaces with non-pathogenic spore-forming bacteria via cleaning products has a favourable effect in reducing the number of pathogenic microorganisms on such surfaces. This decreases the risk of infections and has a beneficial effect on general health. WO 2012150269 and WO 2013153358 describe the use of a probiotic, metabolite or cell lysate for topical treatment of skin conditions, but are limited to lactic acid bacteria and extracts thereof. EP 0975227 describes the use of spores or cell extracts of *Bacillus coagulans, Bacillus subtilis, Bacillus laterosporus* or *Bacillus laevolacticus* in combination with a fructo-oligosaccharide for combating skin infections, but limits these to specific organisms and short sugar chains. WO2011022790 discloses a mixture of Bacillus bacteria which are applied on human skin or animal fur to repel mosquitos. Other patents, such as US 20090035294, are limited to the specific use of specified cell wall components of bacteria for application to the skin. In these cases, living microorganisms or spores thereof are not used.

### Summary of the invention

The invention relates to a composition containing prebiotics and probiotics for application to intact or damaged skin or tissue.

A first aspect of the invention relates to a composition for dermatologic or cosmetic use comprising a mixture of spores of *Bacillus subtilis, Bacillus licheniformis, Bacillus megaterium* and *Bacillus amyloliquefaciens* with a prebiotic sugar.

A second aspect of the invention relates to a combination of a mixture of spores of *Bacillus subtilis, Bacillus licheniformis, Bacillus megaterium* and *Bacillus amyloliquefaciens* with a prebiotic sugar for use in the prevention or treatment of infections of healthy or damaged skin or tissue.

In particular embodiments of the composition or use of the combination, a portion of the aforementioned bacterial spores and/or a portion of the aforementioned sugar is encapsulated in microcapsules.

In particular embodiments of the composition or use of the combination, the prebiotic sugar is inulin.

In particular embodiments of the composition or use of the combination, the aforementioned mixture of bacterial spores is present in a concentration of 1 x 10⁶ to 1 x 10⁹ cfu per gram of the formulation.

In particular embodiments of the composition or use of the combination, the aforementioned prebiotic sugar is present in a concentration of 100 mg to 100 g per kg of the formulation.

In particular embodiments of the composition or use of the combination, the microcapsules comprise a brittle shell filled with a non-aqueous solution and bacterial spores, and can for example have a diameter of 1 to 300 µm.

The synbiotic preparation of the invention is suitable for both human and veterinary use.

Depending on the type of treatment and the body part in question, the composition may be formulated as a liquid, emulsion, cream, ointment, lotion, gel, oil, solution, aerosol spray, powder, or semi-solid formulation.

### Detailed description of the invention

The invention describes a method for the topical treatment of human and animal tissue and skin with a specific mixture of *Bacillus* spores and a prebiotic in the form of a preparation, optionally concentrated in microcapsules for certain applications.

The preparation is characterized by the presence of free *Bacillus* spores, prebiotics, and optionally slow-release microcapsules (according to US 20120076864), and *Bacillus* spores and prebiotics can also be included. This preparation may be a liquid.

A **synbiotic** in the context of the present invention refers to a combination of a probiotic and a prebiotic.

A **probiotic** in the context of the invention refers to spores of a *Bacillus* species or a mixture of spores of different *Bacillus* spp., typically a mixture *of spores of Bacillus subtilis, Bacillus licheniformis, Bacillus megaterium* and *Bacillus amyloliquefaciens.*

In specific embodiments, this mixture is composed solely of spores of *Bacillus subtilis, Bacillus licheniformis, Bacillus megaterium* and *Bacillus amyloliquefaciens.*

In other embodiments, other *Bacillus* spp. may be present. In a specific embodiment, said other *Bacillus* spp. are not *Bacillus coagulans*.

A mixture of the four types *Bacillus subtilis, Bacillus licheniformis, Bacillus megaterium* and *Bacillus amyloliquefaciens* ordinarily contains roughly equal amounts of each of the four types (from 20 to 30%, with a total of 100% for the four types). In some embodiments, this may differ, with at least 10% of each of the four types or 20 to 30% thereof also being present, with a total of 100% for the four types.

The spores should be proportionally distributed in a concentration of 1 x 10⁶ to 1 x 10⁹ per gram of the preparation. On germination, these spores produce a number of enzymes [Boland et al. (2000) Microbiology 146: 57-64] that exert antimicrobial activity by acting on certain cell wall components or harmful microorganisms on the skin and in wound. The transformation of spores to vegetative cells results in absorption of moisture from the surrounding tissue to which the preparation is applied [Knudsen et al. (2015) J. Bacteriol. 19;198(1): 168-77]. This moisture absorption decreases the absolute moisture content of the tissue, thus exerting an inhibitory action on other microorganisms, particularly fungi. The vegetative *Bacillus* cells produce a variety of extracellular enzymes on and in the tissue or wound that reduce the amount of organic nutrients on the skin and in the tissue or wound [Priest (1977) Bacteriol. Rev. 41(3): 711-53]. This reduction exerts an inhibitory action on other microorganisms via nutrient depletion and the general concept of competitive exclusion [Ragione et al. (2003) Vet. Microbiol. 94(3): 245-56]. In addition, the various *Bacillus* cells produce a number of extracellular compounds that exert an inhibitory action via quorum sensing and quorum quenching [Boguslawski et al. (2015) Mol. Microbiol. 96(2): 325-48] on numerous other (potentially harmful) microorganisms on the skin and in the tissue or wound. The vegetative *Bacillus* cells produce bacteriocins [Abriouel et al. (2010) FEMS Microbiol Rev. 35(1): 201-32], which have an inhibitory action on other microorganisms on the skin or in the tissue or the wound. The invention uses *Bacillus subtilis, Bacillus licheniformis, Bacillus megaterium* and *Bacillus amyloliquefaciens* because the combination thereof produces the widest possible variety of bacteriocins, more specifically subclasses 1.1, 1.2 and 1.3 for *Bacillus subtilis,* subclasses 1.3 and 2.3 for *Bacillus licheniformis*, subclass 1.4 for *Bacillus amyloliquefaciens* and class 3 for *Bacillus megaterium*. Moreover, the variety of different *Bacillus* species in the preparation provides the maximum distribution over time (difference in rate of germination) and diversity of the extracellular enzymes and other components produced (such as bacteriocins and quorum sensing molecules and receptors).

**Prebiotic** in the context of the present invention refers to a short-chain sugar (typically 3-9 monosaccharide units), and more specifically one or more galacto-oligosaccharides and/or fructo-oligosaccharides such as inulin. These prebiotic sugars have a twofold effect. On the one hand, they serve as boosters for the probiotic bacteria present in the preparation so that these germinate more rapidly and survive longer by taking in the prebiotic sugars. On the other hand, the prebiotic sugars on the skin or in the tissue provide stimulation of beneficial bacteria already present, such as lactic acid bacteria [Al-Ghazzewi et al. (2014) Benef. Microbes. 5(2): 99-107].

**Microcapsules** in the context of the invention are particles containing a prebiotic and/or a probiotic. The capsules *per se* are described in US 20120076864, and they can optionally be added to the preparation to provide it with longer stability/shelf life and for delayed release of the probiotics and prebiotics on the skin or in the tissue or wound after the free probiotics and prebiotics of the preparation have already exerted their effect. This results in persistent activity of the preparation for up to 6 weeks, provided that sufficient friction is present. If the preparation is applied to the skin, tissue, or a wound that is then covered with a solid plaster cast, the presence of the microcapsules in an established way of ensuring that the preparation exerts a lasting action. In cases where frequent treatment of the skin, tissue, or wound is not possible, it is advantageous to include the microcapsules in the preparation. The microcapsules are ordinarily composed of a brittle shell comprising a liquid containing the spores. The outer layer of this shell can contain functional reactive groups for chemical binding, e.g. to cellulose or other components of bandages and the like. The liquid in the capsules is ordinarily non-aqueous, and more specifically is immiscible with water (e.g. an organic oil, a silicone oil, a fluorocarbon, or mixtures thereof). For example, the shell is composed of a polymer layer, such as a polymer of gelatin, polyurethane, polyolefins, polyamides, polyesters, polysaccharides, silicone resins, epoxy resins, chitosan and aminoplastic resins such as a melamine formaldehyde resin. The microcapsules are ordinarily 1 to 300 µm in diameter. The weight ratio of the shell to the liquid contained therein is typically 1:500 to 1:5000.

The invention describes methods for the treatment of human and animal skin, tissue and wounds with a composition comprising non-pathogenic spore-forming bacteria and prebiotic sugars, optionally with additional microcapsules (that contain these non-pathogenic spore-forming bacteria and/or prebiotic sugars).

This composition can be formulated, for example, as a liquid, emulsion, cream, ointment, lotion, gel, oil, solution, aerosol spray, powder, or semi-solid formulation. Examples of non-pathogenic spore-forming bacteria include *Bacillus subtilis, Bacillus licheniformis, Bacillus megaterium, Bacillus amyloliquefaciens* and optionally *Bacillus polymyxa.*

In typical embodiments, the concentration of these non-pathogenic spore-forming bacteria in the preparation is 1x 10⁸ to 1x 10¹⁰ spores per gram of the preparation, expressed as cfu (colony-forming units). This value refers to the total number of cfu of the above 4 species together (*B. subtilis, B. licheniformis, B. megaterium and B. amyloliquefaciens).*

In typical embodiments, the prebiotic sugars are sugar compounds belonging to the group of oligosaccharides, such as fructo-oligosaccharides and galacto-oligosaccharides.

In typical embodiments, the concentration of the prebiotic sugars in the formulation is 0.5 mg to 100 g per kg of the preparation.

The microcapsules ordinarily comprise a polymer shell that breaks open due to friction and releases the contents of the capsule, and are typically contained in concentrations of 100 mg to 100 g per kg of the preparation.

Bacteria and prebiotic sugars may be present together or separately in a microcapsule. Microcapsules may also be provided containing individual bacterial strains.

In the formulation, up to 10%, 20%, 30%, 50%, or 75% of the prebiotic and/or probiotic may be encapsulated in the microcapsules.

Microcapsules may be dispersed in a liquid or viscous composition. Formulations in which the capsules are attached to a carrier (a patch, bandage, plaster or the like) are also provided.

The treatment can consist of topical washing or spraying of, or application to, skin, tissue, or wounds of humans or animals.

The treatment is used to delay or prevent the growth on the skin, in tissue, or in wounds of bacteria, viruses, yeasts, fungi, or viruses, and can therefore be applied in preventing or combating microbial infections of the skin, tissue, or wounds in humans or animals.

The non-pathogenic spore-forming bacteria can exert an inhibitory action on other microorganisms before, during and after germination, for example by reducing moisture, depleting organic compounds and producing bacteriocins or quorum sensing signal molecules.

The invention can be applied to healthy skin in order to prevent and combat colonization and possible infection by pathogenic bacteria and other microorganisms (such as acne).

The invention can also be applied to wounds, including both disinfected wounds in order to prevent infections and infected wounds in order to combat infections or inhibit the growth of pathogenic bacteria and other microorganisms.

The prebiotic sugars can also have a stimulating action on the growth and activity of non-pathogenic microorganisms already present on the skin, such as Actinobacteria, Firmicutes, Proteobacteria and Bacteroidetes, as well as fungi.

### Examples

### Example 1. Composition of a preparation

An example of a preparation of the invention contains the following amounts per gram:
- 1 x 10⁹ spores of each of the following species: *Bacillus subtilis, Bacillus licheniformis, Bacillus megaterium* and *Bacillus amyloliquefaciens.*
- 10 mg of inulin
- 5 mg of galacto-oligosaccharides
- 10 mg of microcapsules

### Example 2. Formulation of a skin ointment based on the preparation

| | |
|---|---|
| Water | 89.050% |
| Glycerol | 4.000% |
| Caprylic/capric triglyceride | 4.000% |
| Sodium polyacrylate, ethyl hexyl stearate, Trideceth-6 | 1.000% |
| Bacterial culture, non-pathogenic | 1.000% |
| Phenoxyethanol | 0.450% |
| Inulin | 0.100% |
| Galacto-oligosaccharide | 0.050% |
| Ethyl hexyl glycerol | 0.050% |
| Fragrance | 0.300% |

| | |
|---|---|
| [all concentrations indicate percent by weight] | |

The above formulation has been applied on healthy skin of 20 volunteers for up to 24 hours. None of them showed signs of irritation (presence of oedema or of erythema on the skin).

The formulation is applied to various types of intact and damaged skin, tissue, or wounds. The morphology of the skin, tissue, or wound is visually evaluated at regular intervals, and the applied formulation is tested for growth of the *Bacillus* spp. of the formulation and the presence of pathogenic bacteria. This can be carried out, for example, by means of classical bacterial growth and characterization, or by PCR amplification with specific primers followed by denaturing gradient gel electrophoresis (DGGE).

## Claims

1. Composition for dermatologic or cosmetic use, comprising a mixture of *Bacillus subtilis, Bacillus licheniformis, Bacillus megaterium* and *Bacillus amyloliquefaciens* spores with a prebiotic sugar.

2. Combination of:
- a mixture of *Bacillus subtilis, Bacillus licheniformis, Bacillus megaterium* and *Bacillus amyloliquefaciens* spores with
- a prebiotic sugar
for use in the prevention or treatment of infections of healthy or damaged skin or tissue.

3. The composition according to claim 1, or combination for use in the prevention and treatment of intact or damaged skin or tissue according to claim 2, wherein a portion of the aforementioned bacterial spores and a portion of the aforementioned sugar are encapsulated in microcapsules.

4. The composition according to any one of claims 1 through 3, or combination for use in the prevention and treatment of intact or damaged skin or tissue according to claims 2 or 3, wherein the prebiotic sugar is inulin.

5. The composition according to claims 1, 3, or 4, or combination for use in the prevention and treatment of intact or damaged skin or tissue according to claims 2 through 4, in a formulation wherein the aforementioned mixture of bacterial spores is present in a concentration of 1 x 10⁶ to 1 x 10⁹ cfu per of gram of the formulation.

6. The composition according to claims 1 or 3 through 5, or combination for use in the prevention and treatment of intact or damaged skin or tissue according to claims 2 through 5, in a formulation wherein the aforementioned prebiotic sugar is present in a concentration of 100 mg to 100 g per kg of the formulation.

7. The composition according to claims 3 through 6, or combination for use in the prevention and treatment of intact or damaged skin or tissue according to claims 3 through 6, wherein the microcapsule comprises a brittle shell filled with a non-aqueous solution and bacterial spores.

8. The composition according to claim 7, or combination for use in the prevention and treatment of intact or damaged skin or tissue according to claim 7, wherein the diameter of the microcapsules is 1 to 300 µm.

9. Combination for use in the prevention and treatment of intact or damaged skin or tissue according to any of claims 2 through 8 for veterinary use.

10. The composition according to one of claims 1 or 3 through 9, or combination for use in the prevention and treatment of intact or damaged skin or tissue according to claims 2 through 9, formulated as a liquid, emulsion, cream, ointment, lotion, gel, oil, solution, aerosol spray, powder, or semi-solid formulation.

## Patentansprüche

1. Zusammensetzung zur dermatologischen oder kosmetischen Verwendung, umfassend ein Gemisch aus *Bacillus subtilis, Bacillus licheniformis, Bacillus megaterium* und *Bacillus amyloliquefaciens* Sporen mit einem präbiotischen Zucker.

2. Kombination aus:
- einem Gemisch aus *Bacillus subtilis, Bacillus licheniformis, Bacillus megaterium* und *Bacillus amyloliquefaciens* Sporen mit
- einem präbiotischen Zucker
zur Verwendung in der Verhinderung oder Behandlung von Infektionen gesunder oder beschädigter Haut oder gesundem oder beschädigtem Gewebe.

3. Zusammensetzung nach Anspruch 1 oder Kombination zur Verwendung in der Verhinderung oder Behandlung von intakter oder beschädigter Haut oder intaktem oder beschädigtem Gewebe nach Anspruch 2, wobei ein Teil der obengenannten bakteriellen Sporen und ein Teil des obengenannten Zuckers in Mikrokapseln eingekapselt sind.

4. Zusammensetzung nach einem der Ansprüche 1 bis 3 oder Kombination zur Verwendung in der Verhinderung oder Behandlung von intakter oder beschädigter Haut oder intaktem oder beschädigtem Gewebe nach Anspruch 2 oder 3, wobei der präbiotische Zucker Inulin ist.

5. Zusammensetzung nach Ansprüchen 1, 3 oder 4 oder Kombination zur Verwendung in der Verhinderung oder Behandlung von intakter oder beschädigter Haut oder intaktem oder beschädigtem Gewebe nach Ansprüchen 2 bis 4 in einer Formulierung, wobei das obengenannte Gemisch aus bakteriellen Sporen in einer Konzentration von 1 x 10⁶ bis 1 x 10⁹ KBE pro Gramm der Formulierung vorhanden ist.

6. Zusammensetzung nach Ansprüchen 1 oder 3 bis 5 oder Kombination zur Verwendung in der Verhinderung oder Behandlung von intakter oder beschädigter Haut oder intaktem oder beschädigtem Gewebe nach Ansprüchen 2 bis 5 in einer Formulierung, wobei der obengenannte präbiotische Zucker in einer Konzentration von 100 mg bis 100 g pro kg der Formulierung vorhanden ist.

7. Zusammensetzung nach Ansprüchen 3 bis 6 oder Kombination zur Verwendung in der Verhinderung oder Behandlung von intakter oder beschädigter Haut oder intaktem oder beschädigtem Gewebe nach Ansprüchen 3 bis 6, wobei die Mikrokapsel eine brüchige Schale umfasst, die mit einer nicht wässrigen Lösung und bakteriellen Sporen gefüllt ist.

8. Zusammensetzung nach Anspruch 7 oder Kombination zur Verwendung in der Verhinderung oder Behandlung von intakter oder beschädigter Haut oder intaktem oder beschädigtem Gewebe nach Anspruch 7, wobei der Durchmesser der Mikrokapseln 1 bis 300 µm ist.

9. Kombination zur Verwendung in der Verhinderung oder Behandlung von intakter oder beschädigter Haut oder intaktem oder beschädigtem Gewebe nach einem der Ansprüche 2 bis 8 zur Verwendung in der Veterinärmedizin.

10. Zusammensetzung nach einem der Ansprüche 1 oder 3 bis 9 oder Kombination zur Verwendung in der Verhinderung oder Behandlung von intakter oder beschädigter Haut oder intaktem oder beschädigtem Gewebe nach Ansprüchen 2 bis 9, die als Flüssigkeit, Emulsion, Creme, Salbe, Lotion, Gel, Öl, Lösung, Aerosolspray, Pulver oder halbfeste Formulierung formuliert ist.

## Revendications

1. Composition pour un usage dermatologique ou cosmétique, comprenant un mélange de spores de *Bacillus subtilis, Bacillus licheniformis, Bacillus megaterium* et *Bacillus amyloliquefaciens* avec un sucre prébiotique.

2. Combinaison de :
- un mélange de spores de *Bacillus subtilis, Bacillus licheniformis, Bacillus megaterium* et *Bacillus amyloliquefaciens* avec
- un sucre prébiotique
pour un usage dans la prévention ou le traitement d'infections de peau ou tissu sain ou endommagé.

3. Composition selon la revendication 1, ou combinaison pour un usage dans la prévention et le traitement de peau ou tissu intact ou endommagé selon la revendication 2, dans laquelle une portion des spores bactériennes précédemment mentionnées et une portion du sucre précédemment mentionné sont encapsulées dans des microcapsules.

4. Composition selon l'une quelconque des revendications 1 à 3, ou combinaison pour un usage dans la prévention et le traitement de peau ou tissu intact ou endommagé selon les revendications 2 ou 3, dans laquelle le sucre prébiotique est de l'inuline.

5. Composition selon les revendications 1, 3, ou 4, ou combinaison pour un usage dans la prévention et le traitement de peau ou tissu intact ou endommagé selon les revendications 2 à 4, dans une formulation dans laquelle le mélange précédemment mentionné de spores bactériennes est présent en une concentration de 1 × 10⁶ à 1 × 10⁹ ufc par gramme de la formulation.

6. Composition selon les revendications 1 ou 3 à 5, ou combinaison pour un usage dans la prévention et le traitement de peau ou tissu intact ou endommagé selon les revendications 2 à 5, dans une formulation dans laquelle le sucre prébiotique précédemment mentionné est présent en une concentration de 100 mg à 100 g par kg de la formulation.

7. Composition selon les revendications 3 à 6, ou combinaison pour un usage dans la prévention et le traitement de peau ou tissu intact ou endommagé selon les revendications 3 à 6, dans laquelle la microcapsule comprend une enveloppe fragile remplie d'une solution non aqueuse et de spores bactériennes.

8. Composition selon la revendication 7, ou combinaison pour un usage dans la prévention et le traitement de peau ou tissu intact ou endommagé selon la revendication 7, dans laquelle le diamètre des microcapsules est de 1 à 300 pm.

9. Combinaison pour un usage dans la prévention et le traitement de peau ou tissu intact ou endommagé selon l'une quelconque des revendications 2 à 8 pour un usage vétérinaire.

10. Composition selon l'une des revendications 1 ou 3 à 9, ou combinaison pour un usage dans la prévention et le traitement de peau ou tissu intact ou endommagé selon les revendications 2 à 9, formulée comme un liquide, une émulsion, une crème, un onguent, une lotion, un gel, une huile, une solution, une préparation pour aérosol, une poudre, ou une formulation semi-solide.
